# DEMANDE DE BREVET EUROPEEN

(11) **EP 2 692 303 A1**
(43) Date de publication de la demande: **05.02.2014**
(21) Numéro de dépôt: 12178475.5
(22) Date de dépôt: 30.07.2012
(51) Int. Cl.: A61B 17/64

(54) **Mâchoire pour un fixateur externe et articulation utilisant de telles mâchoires**

(71) Demandeur: Swiss Ortho Trauma SA, 1196 Gland (CH)
(72) Inventeur: Bentele, Franz, 34290 Servian (FR)
(74) Mandataire: Leman Consulting S.A.

(57) **Abrégé**

La présente invention concerne une mâchoire (10) pour le maintien de tiges de fixation et/ou de broches d'un fixateur externe. Cette mâchoire comporte au moins deux mors (11', 12') formant un passage (13) prévu pour recevoir la tige de fixation et/ou la broche. La mâchoire est formée d'un premier élément de mâchoire (11) et d'un second élément de mâchoire (12), ces deux éléments de mâchoire étant mobiles l'un par rapport à l'autre le long d'un axe longitudinal (21). L'invention est caractérisée en ce que le premier élément de mâchoire (11) comporte au moins un élément de retenue (17) formé d'une tige (18) et d'une zone élargie (19), en ce que le second élément de mâchoire (12) comporte au moins un logement (25) agencé pour recevoir ledit au moins un élément de retenue (17), et en ce que ledit logement (25) comporte un élément élastique (28) agencé pour coopérer avec ledit élément de retenue (17) lorsque le premier et le second éléments de mâchoire (11, 12) sont assemblés.

La présente invention concerne également une articulation d'un fixateur externe caractérisé en ce qu'il comporte au moins deux mâchoires (10) telles que définies ci-dessus.

## Description

### DOMAINE TECHNIQUE

La présente invention concerne une mâchoire pour le maintien de tiges de fixation et/ou de broches d'un fixateur externe, cette mâchoire comportant au moins deux mors formant un passage prévu pour recevoir la tige de fixation et/ou la broche, la mâchoire étant formée d'un premier élément de mâchoire et d'un second élément de mâchoire, ces deux éléments de mâchoire étant mobiles l'un par rapport à l'autre le long d'un axe longitudinal.

La présente invention concerne également une articulation d'un fixateur externe utilisant des mâchoires telles que définies ci-dessus.

### TECHNIQUE ANTERIEURE

Il existe actuellement différents types de mâchoires utilisées comme fixateur externe dans le domaine de l'orthopédie et/ou de la traumatologie. L'une de ces mâchoires est en particulier décrite dans le brevet européen N° EP 0 700 664. Ce document décrit une articulation destinée à recevoir des tiges et/ou des broches permettant de former une structure rigide pour le maintien d'un os lors de sa réparation après une fracture.

L'articulation décrite dans ce document est formée de deux mâchoires reliées entre elles par un axe et susceptibles de se déplacer le long de cet axe. Les deux mâchoires formant l'articulation sont séparées par un ressort de compression qui a pour effet d'écarter les mâchoires l'une de l'autre le long de l'axe. Ceci permet d'écarter les mors de la mâchoire et donc d'introduire facilement une tige ou une broche dans un passage des mâchoires, en utilisant l'élasticité du ressort.

Cette réalisation présente différents inconvénients. En premier lieu, la réalisation de la mâchoire est relativement complexe. En effet, le ressort est placé dans un logement qu'il est nécessaire de réaliser à cet usage, la forme du logement étant complexe. En second lieu, l'articulation selon l'invention nécessite impérativement la présence de deux mâchoires. En effet, étant donné que le ressort est disposé entre deux mâchoires, l'effet de poussée de l'une des mâchoires en direction de l'autre ne peut être obtenu que dans ce cas. Il en résulte qu'une mâchoire seule ne peut pas être utilisée. L'utilisation d'une seule mâchoire pourrait toutefois être intéressante lorsqu'il est nécessaire de relier deux tiges alignées par exemple. De même, il n'est pas possible d'utiliser plus de deux mâchoires, par exemple trois. Ceci pourrait être intéressant lorsqu'il est souhaitable de créer une structure particulièrement rigide par exemple. Finalement, la mâchoire ne peut pas être démontée et remontée par un utilisateur, ce qui empêche une stérilisation optimale

La demande de brevet US 2006/0255521 décrit une mâchoire pour fixateur externe permettant d'utiliser une seule mâchoire. Pour ceci, un trou borgne est réalisé dans chaque élément de mâchoire de telle façon que ces trous soient en regard l'un de l'autre lorsque la mâchoire est montée. Un élément élastique est placé dans les trous des éléments de mâchoire. Lorsque les éléments de mâchoire doivent être écartés pour permettre l'introduction d'une barre ou d'une broche, cette barre ou broche est pressée en direction des mors. L'élément élastique s'incurve de façon à ce que les mors s'écartent.

En pratique, il est difficile d'introduire un élément élastique dans un trou. Pour que ceci puisse être réalisé, il est nécessaire de prévoir un jeu important entre l'élément élastique et le trou. Il peut alors s'avérer nécessaire de coller l'élément élastique dans le trou de l'une des mâchoires faute de quoi cet élément pourrait tomber lors du démontage de la mâchoire. Lorsque l'élément élastique est collé, la stérilisation de la mâchoire peut être difficile, voire impossible. Dans tous les cas, la mâchoire selon cette invention ne peut pas être démontée et remontée par un utilisateur.

Dans le cas où l'élément élastique n'est pas collé, il peut se déplacer dans les trous. La force qu'il applique aux éléments de mâchoire n'est alors pas constante, ce qui n'est pas souhaitable.

De par le principe de l'invention qui implique que l'élément élastique fonctionne en flexion lorsqu'une tige est introduite et peut coulisser dans au moins l'un des trous lorsque les éléments de mâchoire sont approchés pour fermer l'articulation, il est nécessaire de prévoir un certain jeu des élément de mâchoire autour de la vis de fixation. En effet, en l'absence de jeu, l'élément élastique ne pourrait pas travailler en flexion. Le positionnement de l'un des éléments de mâchoire par rapport à l'autre est alors assuré par la tige ou la broche. En pratique, les broches sont destinées à positionner les os de l'utilisateur et à maintenir la position correcte. Le fait que les éléments de mâchoire ont du jeu l'un par rapport à l'autre implique que le positionnement de l'os ne peut pas être optimal. Du fait de ce jeu, cette mâchoire est peu adaptée à une utilisation comme élément de raccordement de deux broches.

De plus, la solution technique proposée ne permet pas un positionnement angulaire précis entre deux mâchoires et n'assure pas un blocage angulaire mécanique entre ces deux mâchoires.

La présente invention se propose de réaliser une mâchoire plus facile à produire, à monter et à utiliser et qui peut être utilisée soit seule, soit pour former une articulation comportant deux mâchoires, soit pour former une articulation comportant plus de deux mâchoires. La mâchoire peut également être stérilisée. Elle est donc réutilisable. De plus, cette mâchoire ne nécessite pas de jeu avec la vis de fixation; elle permet donc un positionnement précis aussi bien d'un élément de mâchoire par rapport à un autre élément de mâchoire que d'une mâchoire par rapport à une autre mâchoire.

### EXPOSE DE L'INVENTION

Le but de l'invention est atteint par une mâchoire telle que définie en préambule et **caractérisée en ce que** le premier élément de mâchoire comporte au moins un organe de retenue formé d'une tige et d'une zone de retenue, en ce que le deuxième élément de mâchoire comporte au moins un logement agencé pour recevoir ledit au moins un organe de retenue, et en ce que ledit logement comporte un élément élastique agencé pour coopérer avec ledit organe de retenue lorsque le premier et le deuxième élément de mâchoire sont assemblés.

Le but de l'invention est également atteint par une articulation d'un fixateur externe **caractérisée en ce qu'**elle comporte au moins deux mâchoires telles que définies ci-dessus.

La mâchoire selon l'invention est formée de deux éléments de mâchoire qui peuvent se déplacer l'un par rapport à l'autre le long d'un axe longitudinal. En position libre, c'est-à-dire lorsqu'aucune force extérieure n'est appliquée, les deux éléments de mâchoire sont proches l'un de l'autre. Ceci permet de maintenir la tige ou la broche entre deux mors de la mâchoire. Ces deux éléments de mâchoire peuvent être écartés l'un de l'autre de manière à permettre l'introduction d'une tige ou d'une broche sans devoir dévisser une vis, mais simplement en poussant la tige ou la broche dans le passage de la mâchoire.

La mâchoire comporte un élément élastique qui agit sur les deux constituants d'une seule mâchoire. Ainsi, cette mâchoire peut être utilisée aussi bien seule qu'en combinaison avec d'autres mâchoires. Lorsque la tige ou la broche est introduite dans le passage adéquat, elle est maintenue par les mâchoires sans qu'il soit nécessaire qu'une tierce personne la tienne. Ceci permet à une personne seule de réaliser facilement un montage, sans avoir besoin d'aide.

De par la conception de la mâchoire, les éléments élastiques sont relativement simple à mettre en place et à enlever si nécessaire. Ces éléments élastiques sont soumis à une usure ou un vieillissement; ils peuvent être facilement remplacés sans devoir remplacer toute la mâchoire.

Lorsqu'ils sont mis en place, les éléments élastiques sont maintenus mécaniquement, c'est-à-dire qu'il n'est pas nécessaire de les coller. Ceci permet de stériliser la mâchoire sans risquer que les parties élastiques ne sortent de leur emplacement. Il est toutefois également possible de sortir volontairement les éléments élastiques de leur emplacement et plus généralement, de démonter aisément chaque constituant de la mâchoire. Ceci permet par exemple une stérilisation différente entre les éléments élastiques qui sont généralement réalisés en une matière synthétique et le reste de la mâchoire qui est généralement en métal. Par sa conception, les éléments formant la mâchoire se déplacent selon un axe longitudinal lorsqu'ils sont écartés pour laisser passer une tige ou une broche. Ceci permet un positionnent précis des mâchoires et assure un blocage angulaire mécanique entre les éléments de mâchoire.

Selon un mode de réalisation avantageux, les mâchoires comportent des dentures permettant un positionnement angulaire précis et une bonne tenue mécanique.

Les tiges de fixation ou les broches peuvent être mises en place et maintenues dans leur logement même si la vis permettant le blocage final des mâchoires et des articulations n'est pas positionnée. Ceci permet de créer la structure comprenant les articulations, les mâchoires, les tiges de fixation et les broches, puis d'introduire les vis de blocage par la suite. Même avant la mise en place de ces vis, les éléments de mâchoire tiennent en position et il n'y a pas de risque que les mâchoires se démontent ou qu'un élément s'échappe sur le champ opératoire.

### DESCRIPTION SOMMAIRE DES DESSINS

La présente invention et ses avantages seront mieux compris en référence aux figures annexées et à la description détaillée d'un mode de réalisation particulier, dans lesquelles :
- la figure 1 est une vue de profil d'un premier élément de mâchoire selon la présente invention;
- la figure 2 est une vue en perspective de l'élément de mâchoire de la figure 1;
- la figure 3 et une vue en coupe d'un second élément de mâchoire selon la présente invention;
- la figure 4 est une vue en coupe de l'élément de mâchoire de la figure 3 selon une deuxième direction de coupe;
- la figure 5 est une vue de dessus de l'élément de mâchoire des figures 3 et 4;
- la figure 6a est une vue en coupe d'une mâchoire selon l'invention, dans une position d'utilisation ou position fermée;
- la figure 6b est une vue similaire à la figure 6a, dans une position ouverte;
- la figure 7 est une variante de la mâchoire selon l'invention, dans laquelle une tige est introduite dans cette mâchoire;
- la figure 8 est une variante similaire à la figure 7, dans laquelle une broche est introduite dans la mâchoire.
- la figure 9 est une vue en coupe de deux mâchoires formant une articulation, en position d'utilisation;

### MANIERE DE REALISER L'INVENTION

En référence aux figures, une mâchoire 10 selon la présente invention comporte un premier élément de mâchoire 11 pourvu d'un premier mors 11' et un second élément de mâchoire 12 pourvu d'un second mors 12'. Lorsque le premier et le second éléments de mâchoire sont assemblés, les mors 11', 12' définissent un passage 13 destiné à recevoir une tige de fixation ou une broche. Ces éléments de mâchoire comportent un trou traversant 14 destiné à recevoir une vis 15 ou une tige filetée servant à rigidifier la liaison entre les éléments de mâchoire. Plusieurs mâchoires coopérant entre elles forment une articulation, également rigidifiée par la vis 15.

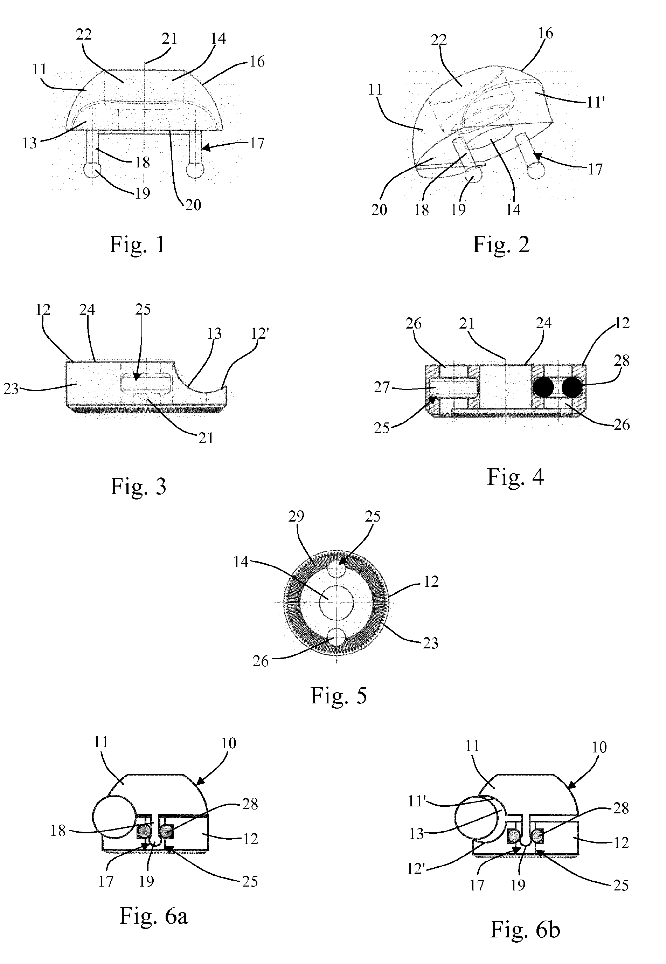

Le premier élément de mâchoire 11 est illustré en détails par les figures 1 et 2. Ce premier élément de mâchoire comporte un corps 16 dans lequel est réalisée une rainure formant le premier mors 11', ainsi que deux éléments de retenue 17. Ces éléments de retenue comportent une tige 18 terminée par une zone élargie 19, formée par exemple d'une portion de sphère. Le corps 16 du premier élément de mâchoire comporte une face sensiblement plane 20, les tiges 18 étant sensiblement perpendiculaires à cette face plane. Les tiges ont un axe longitudinal parallèle à un axe longitudinal 21 du trou traversant 14.

Selon un mode de réalisation avantageux, la face sensiblement plane 20 du premier élément de mâchoire a une enveloppe sensiblement circulaire ou en forme de portion de cercle. Les axes longitudinaux des tiges sont avantageusement placées sur un diamètre de l'enveloppe circulaire. Ces axes longitudinaux sont alignés avec l'axe longitudinal 21 du trou traversant 14, de préférence à équidistance par rapport à cet axe longitudinal 21.

Le corps 16 peut comporter un logement 22 destiné à recevoir une tête de vis 15.

Le second élément de mâchoire 12 est illustré en détail par les figures 3 à 5. Ce second élément de mâchoire comporte un corps 23 ayant une face sensiblement plane 24 dans laquelle est réalisée une rainure formant le second mors 12'. Le passage 13 permet de placer une tige ou une broche dans un plan généralement perpendiculaire à l'axe longitudinal 21 du trou traversant.

Le corps 23 comporte deux logements 25 disposés et dimensionnés de telle façon que lorsque les éléments de mâchoire sont assemblés pour former une mâchoire, les éléments de retenue 17 du premier élément de mâchoire 11 sont disposés dans les logements 25 du second élément de mâchoire 12.

Ces logements sont formés d'un passage 26 traversant généralement le corps 23 et d'une chambre 27 de formes générales cylindriques, le diamètre de la chambre 27 étant supérieur au diamètre du passage, ces deux cylindres étant colinéaires.

Chaque chambre 27 contient un élément élastique 28 généralement formé d'un joint torique.

Le corps 23 peut comporter, à l'opposé de la face plane 24, une zone dentée 29 dont la fonction est expliquée plus bas.

Selon un mode de réalisation, le trou traversant 14 du second élément de mâchoire 12 peut être fileté de façon à permettre d'y visser la vis 15. Selon un autre mode de réalisation, ce trou traversant peut avoir un diamètre plus grand que la vis de façon à ne pas interférer avec son filetage.

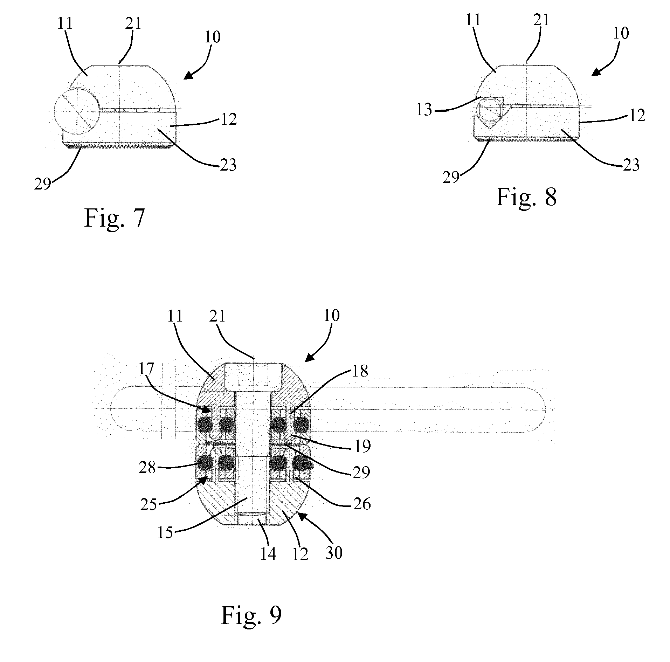

Les figures 6 à 9 illustrent une mâchoire obtenue lorsque les premier et second éléments de mâchoire sont assemblés. Les rainures réalisées dans chacun des éléments de mâchoire sont disposées en regard l'une de l'autre de façon à former le passage 13. Celui-ci a une forme généralement adaptée au type de pièces à maintenir. Ces pièces sont généralement des broches comme cela est illustré par la figure 8 ou des tiges de fixation comme illustré notamment par la figure 9 et ont une section transversale qui peut être circulaire, carrée ou autre. A titre d'exemple, si la tige de fixation à maintenir a une section transversale circulaire, la section transversale du passage 13 est idéalement une portion de cercle formant un angle légèrement supérieur à 180°. De cette façon, un faible écartement des éléments de mâchoire 11, 12 suffit pour introduire la tige de fixation dans le passage. La forme et l'angle de ce passage 13 sont tels qu'une pression de la tige de fixation ou de la broche contre les mors 11', 12', en direction du centre du corps a pour effet d'écarter les deux éléments de mâchoire l'un de l'autre et de permettre la mise en place de cette tige de fixation ou broche dans le passage 13.

Lorsque les éléments de mâchoire sont rapprochés, la tige de fixation est maintenue dans le passage 13. Ceci permet un montage simple de l'ensemble de la structure formée par des tiges de fixation, des broches, des mâchoires et des articulations. Lorsque les mâchoires ou les articulations sont en place, elles sont rigidifiées au moyen de la vis 15 passant par le trou 14 des éléments de mâchoires

La figure 6a représente la mâchoire 10 montée. Dans cette mâchoire, les faces planes 20, 24 des premier et second éléments de mâchoire 11, 12 sont approchées de façon que les éléments de retenue 17 soient positionnés dans les logements 25 et que les rainures soient disposées face à face pour former le passage 13.

La mâchoire peut prendre essentiellement deux positions, à savoir une position fermée représentée par la figure 6a et une position ouverte représentée par la figure 6b.

Dans la position fermée, les éléments élastiques 28 agissent sur la zone élargie 19 des éléments de retenue 17 et maintiennent les deux éléments de mâchoire 11, 12 proche l'un de l'autre. Ceci permet le maintien de la tige de fixation dans le passage 13 de la mâchoire.

La figure 6b représente la mâchoire en position ouverte ou en position de montage. Dans cette position, les deux éléments de mâchoire 11, 12 sont écartés l'un de l'autre. Dans cette position, les éléments de retenue 17 et plus spécifiquement leur zone élargie 19, déforment les éléments élastiques 28. L'élasticité de ces éléments élastiques 28 permet toutefois d'écarter les éléments de mâchoire 11, 12 l'un de l'autre d'une distance suffisante pour permettre le passage de la tige de fixation et sa mise en place dans le passage 13. Lorsque la tige de fixation est introduite dans ce passage, les éléments élastiques 28 agissent sur la zone élargie 19 de l'élément de retenue 17 et ramènent le premier élément de mâchoire 11 à proximité du deuxième élément de mâchoire 12, de façon à fermer cette mâchoire. Le fait d'avoir deux éléments de retenue 17 disposés sur un diamètre des éléments de mâchoire permet un guidage longitudinal de ces éléments de mâchoire l'un par rapport à l'autre. Ceci permet également d'utiliser une vis 15 ayant un diamètre pratiquement égal au trou transversal 14, sans devoir prévoir de jeu. De cette manière, le guidage des éléments est précis et sans jeu.

Les dimensions respectives des logements 25, des chambres 27, des éléments élastiques 28 et des zones élargies 19 sont telles que, en l'absence de contrainte extérieure, les éléments de mâchoire sont maintenus en position fermée. Lorsqu'une contrainte extérieure est appliquée aux éléments de mâchoire de façon à les écarter, ces éléments de mâchoire prennent une position ouverte si la contrainte est inférieure à une valeur de seuil. Les éléments de mâchoire peuvent être séparés l'un de l'autre si la contrainte est supérieure à la valeur de seuil.

La séparation des éléments de mâchoire permet notamment d'accéder aux éléments élastiques 28 en vue de leur remplacement, si ce remplacement est nécessaire ou souhaité. Elle permet également le retrait des éléments élastiques de façon à permettre une stérilisation séparée des éléments métalliques c'est-à-dire notamment les corps de la mâchoire, et des éléments en matière synthétique, c'est-à-dire les éléments élastiques.

La figure 9 représente une articulation 30 formée de deux mâchoires 10 et d'une vis 15.

Comme indiqué précédemment, le premier élément de mâchoire de chaque mâchoire peut comporter une zone dentée 29. Deux zones dentées peuvent coopérer de façon à assurer un blocage des deux mâchoires l'une par rapport à l'autre dans une position angulaire choisie et assurer ainsi un positionnement angulaire précis. Il est également possible de prévoir des zones dentées sur le deuxième élément de mâchoire, ce qui peut être utile lorsque plus de deux mâchoires sont utilisées sur un même axe.

Lorsque deux mâchoires ou plus sont utilisées simultanément pour former une articulation, le fonctionnement de chacune d'entre elles est indépendant de l'autre mâchoire. La vis 15 doit être adaptée au nombre de mâchoires formant l'articulation.

La vis peut être utilisée avec un écrou (non représenté) pour bloquer le fixateur externe lorsque toutes les tiges et broches ont été mises en place dans la position souhaitée. Il est également possible de prévoir une zone filetée dans l'élément de mâchoire le plus éloigné de celui contenant le logement 22 prévu pour la tête de vis et un trou de diamètre supérieur au filetage dans les autres éléments de mâchoire. Dans ce cas, la vis peut être bloquée dans la zone filetée sans utiliser un écrou.

La mâchoire selon la présente invention permet d'introduire et de retirer aisément et rapidement des tiges et des broches d'un fixateur externe. Ainsi, lorsque les broches sont positionnées dans le ou les os à maintenir, la structure du fixateur externe peut être rapidement mise en place, sans nécessiter l'aide de plusieurs personnes.

La mâchoire peut être utilisée en combinaison avec une autre mâchoire similaire pour former une articulation. Cette articulation peut être formée de deux mâchoires ou plus. La mâchoire peut également être utilisée seule. La mâchoire et l'articulation de l'invention offrent donc une grande souplesse et une efficacité accrue par rapport aux dispositifs connus.

## Revendications

1. Mâchoire pour le maintien de tiges de fixation et/ou de broches d'un fixateur externe, cette mâchoire comportant au moins deux mors formant un passage prévu pour recevoir la tige de fixation et/ou la broche, la mâchoire étant formée d'un premier élément de mâchoire et d'un second élément de mâchoire, ces deux éléments de mâchoire étant mobiles l'un par rapport à l'autre le long d'un axe longitudinal, **caractérisée en ce que** le premier élément de mâchoire (11) comporte au moins un élément de retenue (17) formé d'une tige (18) et d'une zone élargie (19), **en ce que** le second élément de mâchoire (12) comporte au moins un logement (25) agencé pour recevoir ledit au moins un élément de retenue (17), et **en ce que** ledit logement (25) comporte un élément élastique (28) agencé pour coopérer avec ledit élément de retenue (17) lorsque le premier et le second éléments de mâchoire (11, 12) sont assemblés.

2. Mâchoire selon la revendication 1, **caractérisée en ce que** ledit élément élastique (28) comporte un joint torique.

3. Mâchoire selon la revendication 2, **caractérisée en ce que** ledit joint torique est disposé autour de la tige (18) de l'élément de retenue (17) lorsque le premier et le second éléments de mâchoire (11, 12) sont assemblés.

4. Mâchoire selon la revendication 1, **caractérisée en ce qu'**elle comporte deux éléments de retenue (17) et deux logements (25).

5. Mâchoire selon la revendication 1, **caractérisée en ce que** les éléments de mâchoire ont une face (20, 24) ayant une enveloppe circulaire et **en ce que** lesdits éléments de retenue (17) et lesdits logements (25) sont disposés sur un diamètre de ladite enveloppe circulaire.

6. Mâchoire selon la revendication 1, **caractérisée en ce que** chaque logement (25) comporte une chambre (27) dans laquelle l'élément élastique (28) est au moins partiellement disposé.

7. Mâchoire selon la revendication 1, **caractérisée en ce qu'**au moins l'une des faces d'au moins l'un des éléments de mâchoire (11, 12) comporte une zone dentée (29).

8. Mâchoire selon la revendication 1, **caractérisée en ce qu'**elle comporte des moyens de blocage d'un élément de mâchoire par rapport à l'autre.

9. Mâchoire selon la revendication 1, **caractérisée en ce que** les moyens de blocage comportent une vis (15).

10. Articulation d'un fixateur externe **caractérisé en ce qu'il** comporte au moins deux mâchoires (10) telles que définies dans l'une quelconque des revendications précédentes.

11. Articulation selon la revendication 10, **caractérisé en ce que** chaque mâchoire (10) comporte une zone dentée (29), les zones dentées de deux mâchoires adjacentes étant disposées en regard l'une de l'autre.

12. Articulation selon la revendication 10 ou 11, **caractérisé en ce qu'il** comporte des moyens de blocage d'une mâchoire par rapport à l'autre.

13. Articulation selon la revendication 12, **caractérisé en ce que** les moyens de blocage comportent une vis (15) passant par un trou (14) traversant lesdites mâchoires.
